# EUROPEAN PATENT SPECIFICATION

(11) **EP 3 847 795 B1**
(45) Date of publication and mention of the grant of the patent: **05.11.2025**
(21) Application number: 19766107.7
(22) Date of filing: 04.09.2019
(51) Int. Cl.: A61B 5/16, G06F 3/01, G06F 3/03, G06F 1/16, G16H 50/20, A61B 5/00, H04L 67/12, A61B 5/11, G16H 20/70, G16H 40/63

(54) **APPARATUS AND PROGRAM FOR DETERMINING A COGNITIVE STATE OF A USER OF A MOBILE DEVICE**
EINRICHTUNG UND PROGRAMM ZUR BESTIMMUNG EINES KOGNITIVEN ZUSTANDS EINES BENUTZERS EINER MOBILEN VORRICHTUNG
APPAREIL ET PROGRAMME PERMETTANT DE DÉTERMINER UN ÉTAT COGNITIF D'UN UTILISATEUR D'UN DISPOSITIF MOBILE

(30) Priority: 05.09.2018 EP 18192686; 15.05.2019 EP 19174607
(43) Date of publication of application: 14.07.2021
(73) Proprietor: Altoida Inc., Washington, DC 20003 (US)
(72) Inventor: TARNANAS, Ioannis, Houston, TX 77055 (US); BÜGLER, Maximilian, 83135 Schechen (DE)
(74) Representative: Williams Powell
(86) International application number: PCT/IB2019/057436
(87) International publication number: WO 2020/049470

(56) References cited:
- WO-A1-2017/127571
- WO-A1-2018/081134
- US-A1- 2017 251 945
- US-B1- 9 883 831
- ANONYMOUS: "Apple unveils its 2017 iPhones, including the premium iPhone X", 12 September 2017 (2017-09-12), pages 1 - 13, XP093008158, Retrieved from the Internet <URL:https://newatlas.com/apple-iphone-x/51315/> [retrieved on 20221214]

## Description

### Field of the invention

The present invention concerns an apparatus and computer program for determining a cognitive state of a user of a mobile device.

### Description of related art

The detection of cognitive states, in particular of health states with respect to brain diseases or brain damages, are difficult to detect. A specialized doctor is needed which knows the symptoms of the respective brain disease or brain damage. Even then, the detection of such health states by human doctors is not very objective and often error-prone.

WO2018081134 discloses apparatus for using data collected from the responses of an individual with computerized tasks of a cognitive platform to derive performance metrics as an indicator of cognitive abilities, and applying predictive models to generate an indication of neurodegenerative condition. The apparatus may employ a variety of different devices including a smartphone, a tablet, a slate, an electronic-reader (e-reader), a digital assistant, or other electronic reader or hand-held, portable, or wearable computing device, or any other equivalent device, a gaming console, or other computing system that can be used to render game-like elements, a head-mounted device, such as smart eyeglasses with built-in displays, a smart goggle with built-in displays, or a smart helmet with built- in displays. In some example implementations, the system may be stationary, such as a desktop computing system that includes a main computer and a desktop display (or a projector display), in which the user provides inputs to an App program using a keyboard, a computer mouse, a joystick, handheld consoles, wristbands, or other wearable devices having sensors that communicate with the main computer using wired or wireless communication.

Recently, a mobile device with a position sensor was suggested to detect the health state based on the path of the mobile device hold by the user. This worked surprisingly well and objective to detect a cognitive state of the user.

### Brief summary of the invention

It is the object of the invention to further improve the mobile device for detecting the cognitive state.

This object is solved by an apparatus, and a computer program according to the independent claims.

The use of user features recorded with a second camera of the mobile device in combination with the path of the mobile device significantly improves the detection quality for the cognitive state.

According to an aspect of the present invention, there is provided apparatus for determining a cognitive state of a user of a mobile device as specified in claim 1
According to another aspect of the present invention, there is provided a computer program as specified in claim 14.

In one embodiment, the at least one user feature comprises at least one facial feature of the user. The facial features of the user work particular among user features detected in the images captured by the second camera.

In one embodiment, the further camera is configured to take a series of images while the user solves the task, wherein the user feature means is configured to detect the at least one feature of the user in the series of images and to determine a time series of the at least one feature detected in the series of images, wherein the cognitive state means is configured to determine the cognitive state of user further based on the time series of the at least one feature of the user.

In one embodiment, a sampling window comprises a plurality of sampling steps over time, wherein the path means is configured record in each sampling step at least one position of the mobile device, wherein the further camera is configured to record at least one image in each sampling step, wherein the user feature means is configured to detect for each sampling step the at least one feature of the user in the at least one image of recorded in the sampling step by the further camera, wherein the cognitive state means is configured to determine the cognitive state of user based on a time series in the sampling window comprising for each sampling step a sampling point based on the at least one feature of the user detected in this sampling step and based on the at least one position of the mobile device determined in this sampling step.

In one embodiment, the sampling window corresponds to time window in which the mobile device is located closer to the at least one target position than a threshold and/or which corresponds to a certain time period or path length before the at least one target position is considered to be found. Preferably, the sampling window comprises a plurality of sub windows, wherein each sub window corresponds to (only) one defined target position, wherein each sub window corresponds to time window in which the mobile device is located closer to the target position corresponding to the respective sub window than a threshold and/or which corresponds to a certain time period or path length before the target position corresponding to the respective sub window is considered to be found. The threshold distance from the target position is preferably less than 5 meters (m), preferably less than 4 m, preferably less than 3 m. The threshold distance from the target position is preferably more than 0,5 meters (m), preferably more than 1 m.

In one embodiment, the optical output means comprises a display arranged on a first side of the mobile device, wherein the further camera is arranged on the first side of the mobile device and/or is arranged such that the second view direction shows in the same direction as the normal vector of surface of the display.

In one embodiment, the camera is arranged on a second side of the mobile device opposed to the first side and/or is arranged such that the first view direction shows in the opposite direction as the normal vector of surface of the display.

In one embodiment, the mobile device is a portable handheld device.

In one embodiment, the task means is configured to define at least one target position in the augmented reality environment and to perform the following steps for each of the at least one target position: invite the user to find the respective target position in the augmented reality environment; consider the respective target position as found in the augmented reality environment, when the user has moved the mobile device in a position in the environment, in which the at least one target position is captured by the camera and is shown, maybe under certain conditions, on the optical output means.

In one embodiment, the task means is configured to define the at least one target position in the augmented reality environment based on a user input of the mobile device.

In one embodiment, the at least one target position comprises at least two target positions, wherein the task means is configured to associate each of the at least two target positions with a different feature which is output to the user by the output means, when the respective target position is defined and/or shown in the augmented reality environment, wherein the user is invited to find one of the at least two target positions in the augmented reality environment by outputting the feature associated with the one target position by the output means, and /or to subsequently invite the user to find the at least two target positions such that after one of the at least two target positions is considered to be found, the user is invited to find a subsequent one of the at least two target positions.

In one embodiment, the task means is configured to give out via the output means a plurality of stimuli for distracting the user while solving the task and/or while finding each of the at least one target positions.

In one embodiment, the task means is configured to require the user after each stimulus to solve a sub-task before continuing with solving the task and/or with finding the respective target position invited to find.

In one embodiment, the apparatus comprising a user input means, wherein the task means is configured to consider the sub-task solved, when receiving a certain user input over the user input means.

In one embodiment, the cognitive state is a health state with respect to at least one brain disease or brain damage

### Brief Description of the Drawings

The invention will be better understood with the aid of the description of an embodiment given by way of example and illustrated by the figures, in which:
Fig. 1 shows a schematic view of an embodiment of the apparatus for determining a cognitive state of the user.
Fig. 2 shows a schematic view of an embodiment of the method for determining a cognitive state of the user
Fig. 3 shows a schematic view of an embodiment of the apparatus for determining machine learning data.

### Detailed Description of possible embodiments of the Invention

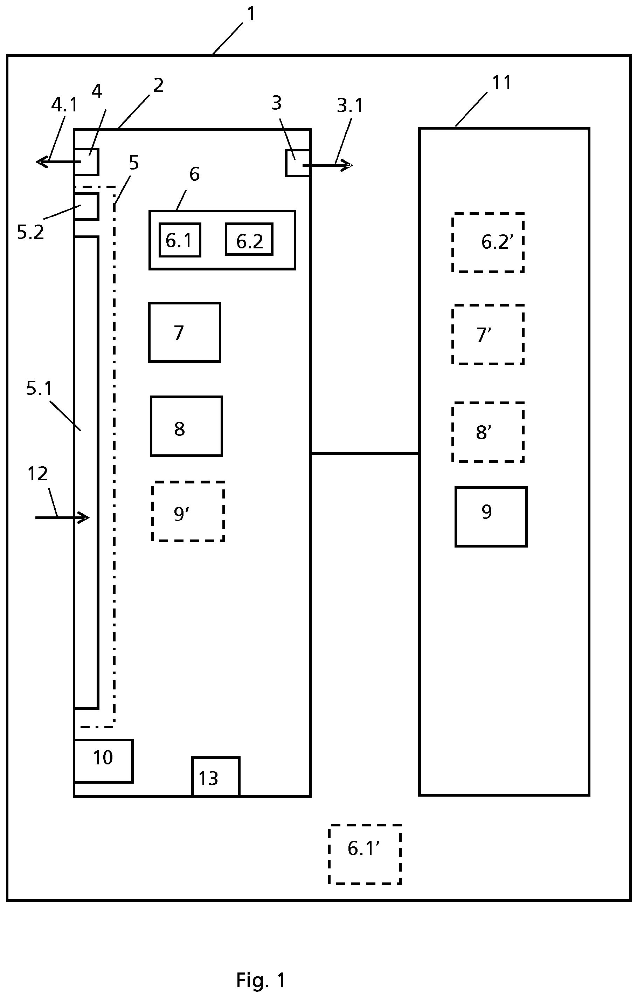

Fig. 1 shows an embodiment of the apparatus 1 for determining a cognitive state of the user. The apparatus 1 comprises a mobile device 2, a first camera 3 in the mobile device 2, a second camera 4 in the mobile device 2, an output means 5 in the mobile device 2, a path means 6, a task means 7, a user feature means 8, a cognitive state means 9, preferably a user input means 10 and a microphone 13.

The mobile device 2 is preferably a handheld portable device like a smartphone. However, the mobile device 2 can also be any other device portable by the user. For example the device can be a wearable device, for example a (smart) bracelet like a smart watch, (smart) glasses like google glasses (registered trademark) or any other device wearable by the user. The mobile device 2 can be realized in one single device or also be realized in at least two individual devices. The at least two individual devices can for example be at least two of a portable handheld device (e.g. smartphone), a bracelet (e.g. a smart bracelet like a smart watch), glasses (e.g. augmented reality (AR) glasses or any other device wearable by the user. The mobile device 2, in particular the handheld portable device comprises a first side and a second side opposite to the first side. If the mobile device 2 comprises at least two individual devices, the first side and the opposed second side is arranged on the same individual device. However, it is also possible that the first side is on a first individual device and the second side on a second individual device.

The apparatus 1 could comprise a further device 11 for realising some computational work remote from the mobile device 2. The further device 11 could be connected with the mobile device 2 to exchange data and/or results. The connection between the mobile device 2 and the further device 11 is preferably via internet, but could be also via WLAN, mobile phone network, other wireless communication protocols and/or other communication techniques. The further device 11 is for example a server, preferably an internet connected server and/or preferably remote from the mobile device 2. However, the further device 11 could be also arranged in the environment of the mobile device 2 and/or connected e.g. via WLAN and/or LAN and/or other wireless communication protocols. However, the further device 11 is optional and is not needed, if all computational work is done in the mobile device 2. In this case, the apparatus 1 corresponds to the mobile device 2.

The output means 5 is configured to output information to the user of the mobile device 2.

The output means 5 comprises preferably an optical output means 5 for showing the user of the mobile device 2 optical information. The optical output means 5 is preferably a display. The optical output means 5.1, in particular the display is preferably arranged on a second side of the mobile device 2, in particular of the handheld portable device. However, other optical output means 5.1 like a projector (e.g. an eye projector in glasses) are possible. The optical output means 5.1 are arranged such in the mobile device 2 that the user can watch the information from the optical output means 5.1 in an output view direction, i.e. the direction from the user to the optical output means 5.1 allowing the user to watch the information from the optical output means 5.1.

In one embodiment, the output means 5 comprises further audio output means 5.2 like a loudspeaker for giving out information to the user.

The input means 10 is configured to input user commands into the mobile device 2. The input means 10 is preferably arranged in the mobile device 2. Preferably, the input means 10 comprises an indicator means for selecting a position on the optical output means 5.1. The indicator means is preferably a touch sensor which is preferably arranged over the display of the mobile device 2, i.e. the display and the touch sensor constitute a touch screen. The indicator means could however be mouse, trackball, knob or any other indicator means. The indicator means could be also a hand or eye direction recognition which indicates a position in the AR environment. The input means 10 could however also comprise a microphone (e.g. for voice commands) or any other input means. The microphone of the input means 10 could correspond to the microphone 13 described later. The input means 10 is preferred, but is not essential for the invention.

The first camera 3 and/or the second camera 4 can be any suitable sensor for recording images of the environment, preferably of features of the environment visible to the eye of the user. Preferably, the first camera 3 and/or the second camera 4 is/are an optical camera for recording images copying the environment as seen by the user. However, the first camera 3 and/or the second camera 4 can also be any other camera, e.g. 3D camera, a time of flight camera, or an optical camera covering other wavelengths then of the visible spectrum, e.g. an infrared camera or an ultraviolet camera, etc. Preferably, the first camera 3 and/or the second camera 4 comprise a digital image sensor and/or a (optical) lens (for focusing the image on the digital image sensor).

The first camera 3 is arranged such in the mobile device 2 that images of the environment of the mobile device 2 are recorded in a first view direction 3.1 and/or such that the images of the first camera 3 can be shown on the optical output means 5.1 for AR purposes and/or such that, when the user looks on the optical output means 5.1 for watching the optical information of the optical output means 5.1 and an image currently recorded with the first camera 3 is shown on the optical output means 5.1, the user sees on the optical output means 5.1 the image of the environment behind the optical output means 5.1. This environment of the mobile device 2 captured by the first camera 3 and replayed (in real time) by the optical output means 5.1 is defined here as AR environment. This AR environment can be augmented by other information or not and shall thus not be limited to the digitalised environment with augmented information.

The task means 7 is configured to interact with the user of the mobile device 2 (via the mobile device 2) to make the user move the mobile device 2 in the environment of the mobile device 2. Preferably, the task means 7 is configured to interact with the user of the mobile device 2 (via the mobile device 2) to make the user walk with the mobile device 2 in the environment of the mobile device 2. The task means 7 is configured to interact with the user of the mobile device 2 (via the mobile device 2) by giving the user a task to solve which makes the user move (in particular walk with) the mobile device 2 in the environment of the mobile device 2. In one embodiment, the task means 7 is configured to use the AR environment of the mobile device 2 in order to make the user solve the task and/or to make the user move (in particular walk with) the mobile device 2 in the environment.

In one embodiment, this task is to search or find at least one defined target position in the (AR) environment. Preferably, the task is that the user needs to move the mobile device 2 (in particular walk with the mobile device 2) to find the at least one defined target position in the AR environment. In other words, the user needs to move (in particular walk with) the mobile device 2 in the environment to the at least one defined target position in the environment and to direct the first camera 3 on the at least one defined target position (so that the defined target position appears on the optical output means 5.1). Once one of the at least one defined target position appears (maybe with at least one condition) on the optical output means 5.1 and/or appears (maybe with at least one condition) in the AR environment, the one defined target position is considered as found. The at least one condition is for example that the mobile device 2 and/or the first camera 3 is closer than a threshold vicinity to the defined target position and/or defined target position is recorded with the first camera 3 in a certain angular view range and/or the currently recorded image of the defined target position corresponds to an image recorded, when the target position was defined. Many other conditions are possible for checking, if the defined target position is found. If the at least one defined target position comprises a first defined target position and a second defined target position, the task means 7 is configured to interact with the user of the mobile device 2 to make the user find the first defined target position. Once the user has found the first defined target position, the task means 7 is configured to interact with the user of the mobile device 2 to make the user find the second defined target position. Thus, the task means 7 is configured to interact with the user of the mobile device 2 to make the user find subsequently each of the at least two defined target positions. Once all of the at least one defined target position has been found, the task is considered as solved.

Preferably, each of the at least one defined target position is shown in the AR environment to the user and/or is defined in the AR environment by the user, such that the user can memorize the at least one target position. After the user has memorized (all of) the at least one target position, the user is invited to find (all of) the at least one defined target positions, preferably one after another. Thus, in a first phase (all of) the at least one defined target positions are shown to the user or are defined by the user, and in a second phase, following after the first phase, all of the at least one defined target positions are searched as described above.

Preferably, each of the at least one defined target position is shown in the AR environment to the user, such that the user can memorize the at least one target position. This can be done simultaneously or subsequently. After the user has seen all of the at least one defined target position and/or has memorized them, the user is invited to find all of the at least one defined target positions, preferably one after another. Thus, in a first phase (all of) the at least one defined target positions are shown to the user, and in a second phase, following after the first phase, all of the at least one defined target positions are searched as described above. Preferably, each at least one defined target position is associated with a visualization object which is shown to the user at the defined target position in the AR environment (in the first phase) so that the user can associate the visualization object to the associated defined target position. Preferably, in the second phase, the user is invited to search one of the at least one defined target positions by showing on the optical output means 5.1 or otherwise indicating via the output means 5 the visualization object associated with this one of the at least one defined target position. Preferably, in the second phase, the visualization object is shown without any indication of the associated defined target position so that the user needs to remember the associated defined target position and find it with the user's mobile device 2. Preferably, the task means 7 is configured to be environment independent, i.e. the user can perform the task in any environment he is currently situated. There is no special environment necessary to perform the tasks.

Preferably, the at least one target position is defined by the user via the input means 10, preferably in the first phase. Preferably, the at least one target position is defined by the user by receiving via the indicating means from the user an indication of a target position in the AR environment. However, the mobile device 2 can also receive any other input via the input means 10 which defines the at least one target position. For example, the target position can be defined by the task means 7 (e.g. randomly) in the AR environment currently shown in the optical output means 5.1, when receiving an input of the user via the input means 10. Preferably, each of the at least one target position is shown to the user, when defined by the user, e.g. by shown the associated visualization device in the AR environment.

The task means 7 is preferably configured to interact via the mobile phone 2 with the user to make the user solve sub-tasks while the user is solving the task, in particular while the user finds each of the at least one defined target position. The task means 7 is preferably configured to invite the user to solve a sub-task in the average at least every 10 seconds (sec), preferably at least every 8 sec, preferably at least every 5 sec, preferably at least every 3 sec, preferably at least every 2 sec, while the user is solving the task, in particular while the user finds each of the at least one defined target position. The invitation for the sub-task can be given out periodically or randomly. The sub-task should distract the user from solving the task. Preferably, the sub-task is a tactile interaction with a touch screen of the mobile device 2. One example of a sub-task could be to activate a button of the mobile device 2, when the task means 7 invites the user to solve the sub-task. Preferably, the button is a soft button shown on the optical output means 5.1. Preferably, the sub-task is solved, if the user indicates by the indication means of the input means 10 the soft button shown on the optical output means 5.2. This distracts the user from the task, because the user has to move his focus from the (AR) environment to the button and activate the button by the indication means. The sub-task of activating a button is just an example. The user could also say a voice command recorded by the microphone of the input means 10. The invitation to solve the sub-task could be for example by an audio signal output via the audio output means 5.2. However, other invitations are possible like a haptic signal (e.g. by a vibration of the mobile device 2). The combination of task and sub-task is particularly advantageous, because the task makes the user move (in particular walk with) the mobile device 2 in the environment, while the sub-tasks distract the user from the task and thus from the movement he is doing. This combination of movement and distraction is analysed later to analyse the cognitive state of the user.

The task means 7 is preferably arranged in the mobile device 2. However, it would also be possible to arrange the task means 7' completely or partly outside of the mobile device 2, e.g. in the further device 11.

The path means 6 is configured to determine the path of the mobile device 2, in particular the path which the mobile device 2 follows, while at least a part of the task is solved. The path is a sequence of position points (in a position sampling window). A position point comprises a one-, two- or preferably three-dimensional location and/or a one-, two- or preferably three-dimensional orientation of the mobile device 2. Thus, a position point can have between one and six dimensions. Preferably, the position point comprises at least the three-dimensional location. The position, i.e. the location and/or orientation, can be absolute, i.e. measured with respect to a known position, or relative, i.e. measured with respect to an unknown position (e.g. the first position point measured or the previous position point measured respectively for each time point). The relative position can also relate to the deviation position with respect to the last measured sampling/position or an inertial acceleration point (with respect to a translation or a rotation) as usually done in an accelerometer or gyroscope.

The path means 6 comprises preferably a position sensor 6.1. The position sensor 6.1 is configured to measure the position points of the mobile device 2. The position sensor 6.1 is preferably configured to measure a series of position points in a position sampling window. The position sampling window corresponds to a time window in which at least a part of the task is solved. The time points, when the position points are recorded in the position sampling window, are called position sampling points. The position sampling points can be measured with a position sampling frequency and/or periodically with constant position sampling steps. A position point gives thus the position of the mobile device 2 at the position sampling point. Preferably, the position sensor 6.1 is an inertial measurement unit (IMU) for detecting the positional change of the mobile device 2. The position sensor 6.1 and/or IMU comprises preferably an accelerometer (for measuring the locational change of the mobile device 2) and/or a gyroscope (for measuring the orientation change of the mobile device 2). The IMU measurements can be transformed in position sample points or can be used directly as the incremental acceleration points as measured by the IMU. Preferably, the position sensor 6.1 and/or the IMU comprises a magnetometer for detecting the absolute orientation of the mobile device 2 (based on the magnetic field of the earth). However, the position sensor 6.1 can comprise additionally or alternatively other means for detecting the position. For example, a triangulation sensor. The triangulation sensor could measure the position based on different signals sent from satellites (e.g. GPS) or from cell phone base stations. It is further possible that the position sensor 6.1 is realized by the first camera 3. Preferably, the position points are determined by a combination of the IMU and the first camera 3. The position sensor 6.1 is preferably arranged in the mobile device 2. However, it would also be possible to arrange the position sensor 6.1' completely or partly outside of the mobile device 2, e.g. a sensor detecting the position based on signals sent out from the mobile device 2.

The path means 6 comprises preferably a path means. The path means 6.2 is configured to determine a path based on the measurements of the position sensor 6.1. Preferably, the path means 6.2 is arranged in the mobile device 2. The path means 6.2 can be realized in a specialised chip (e.g. in the position sensor 6.1) and/or in the Central Processing Unit (CPU) and/or in any other processing unit in the mobile device 2. It is however also possible to arrange the path means 6.2 completely or partly outside of the mobile device 2, e.g. in the server 11 or in the same device of the position sensor 6.1'.

The mobile device 2 comprises a second camera 4 and/or a microphone 13.

The second camera 4 points in a second view direction 4.1 such that images in a second view direction 4.1 are recorded. The second view direction 4.1 and/or the orientation of the second camera 4 is such that the second view direction 4.1 or said orientation is opposed to the first view direction 3.1 (preferably parallel) and/or in the same direction as the output view direction 12 of the optical output means and/or such that images recorded by the second camera 4 point in a direction in which the user, in particular the user's face is located, when he/she looks into the optical output means 5.1. In other words, the second camera 4 captures the user while he/she is in the position of watching the information output by the optical output means 5.1. The second camera 4 is configured to record images with the user, while the user solves (at least a part of) the task with the mobile device 2. The second camera 4 is preferably configured to measure a series of images in an image sampling window. The image sampling window corresponds to a time window in which (at least a part of) the task is solved. The time points, when the images are recorded in the image sampling window, are called image sampling points. The image sampling points can be measured with an image sampling frequency and/or periodically with constant image sampling steps between two subsequent image sampling points. An image gives thus the image of the mobile device 2 at the image sampling point.

The microphone 13 is configured to record an audio signal of the environment of the mobile device 2. The microphone 13 is configured to record the audio signal of the user, while the user solves (at least a part of) the task with the mobile device 2. The microphone 13 is preferably configured to measure a series of audio points in an audio sampling window. The audio sampling window corresponds to a time window in which (at least a part of) the task is solved. The time points, when the audio points are recorded in the audio sampling window, are called audio sampling points. The audio sampling points can be measured with an audio sampling frequency and/or periodically with constant audio sampling steps between two subsequent audio sampling points. An audio point represents thus the sound around the mobile device 2 at the audio sampling point.

The position sampling window, the image sampling window and/or the audio sampling window are preferably identical time windows and/or (only) partly overlapping time windows. However, in a less preferred embodiment, it is also possible that the position sampling window, the image sampling window and/or the audio sampling window do not overlap, i.e. the measurements come from different time windows. The position sampling window, the image sampling window and/or the audio sampling window correspond preferably to an uninterrupted time window. However, it is also possible to use a time window with sampling breaks. The position sampling frequency, the image sampling frequency and/or the audio sampling frequency can be the same or be different. The position sampling points, the image sampling points and/or the audio sampling points can be contemporaneous or at different points in time. The position sampling window, the image sampling window and/or the audio sampling window correspond preferably to a time window in which the user/mobile device 2 is closer to the at least one defined target position than a defined threshold and/or to a time window which corresponds to a certain time period or path length before the user finds each of the at least one target position. **If** the task comprises multiple defined target positions, the position sampling window, the image sampling window and/or the audio sampling window correspond preferably to a concatenation of sub time windows corresponding each to one of the defined target positions, i.e. each sub time window corresponds to a time window in which the user/mobile device 2 is closer to the defined target position corresponding to this sub time window than a defined threshold and/or to a time window which corresponds to a certain time period or path length before the user finds the target position corresponding to the sub time window.

The user feature means 8 is configured to determine at least one user feature based on the images recorded by the further camera 4 while the user solves the task and/or in the audio signal recorded by the microphone 13 while the user solves the task.

The at least one user feature determined based on the images recorded by the further camera 4 is preferably at least one facial feature of the user. Therefore, the user feature means 8 is configured to detect a face of the user in each of at least some of the images recorded, while the user solves the task, and to detect the at least one facial feature from the detected face of the image. Examples of facial features could be a pupil dilation, a mouth form, the eye opening state, eye brown state, eye looking direction etc. However, it is possible to detect other user features from the images recorded. The at least one user feature determined based on the images recorded by the second camera 4 is preferably a movement of at least one visible feature of the user, preferably of at least one facial feature of the user. Therefore, the user feature means 8 is configured to detect a body, in particular a face of the user in each of at least some of the images recorded, while the user solves the task, and to detect the movement of at least one body or facial feature from the detected face of the images. In a preferred embodiment, the pupil dilation is used as a user feature extracted from the images recorded by the second camera 4. The size of the pupil(s) of the user, when solving the task, is extracted from the images taken with the second camera 4, when the user solves the task (resulting in a time series of the pupil size(s) during the sample window, when the user solves the task). The pupil size can comprise the pupil size of one eye (one dimensional) or of both eyes (two-dimensional) or of a combination of the pupil sizes of both eyes (one-dimensional) or other measures derived from the pupil size of one or two eyes of the user. The pupil size can be normalized with respect to a reference pupil size of the user. The reference pupil size can be for example an average pupil size of the user, e.g. the average pupil size during a time period when the task is not cognitive demanding for the user. The normalized pupil size and/or a variation of the normalized pupil size could be used for example as a user feature for the pupil dilation.

The at least one user feature determined based on the audio signal recorded by the microphone is preferably is preferably a sound produced by the mouth or nose of the user. This could be for example a feature extracted from the talk (speech) of the user. The task could for example cause the user to talk. In another example, this could be for example a breathing. However, it is possible to detect other user features from the audio signal recorded. In a preferred embodiment, the regularity of speech is used a user feature. The regularity of speech can be measured by measuring one or a combination of the following speech parameters: amplitude or power of sound of speech (for certain frequencies or for all frequencies), velocity of speech, pauses between words, intonation, etc.. Then, the speech parameter(s) is/are normalized to their reference speech parameter(s) of the user. The reference speech parameter(s) can be for example an average speech parameter of the user, e.g. the average speech parameter(s) during a time period when the task is not cognitive demanding for the user. The one or the combination of normalized speech parameters or the variation/fluctuation of the one or the combination of normalized speech parameters can be used a user feature.

The at least one user feature can be determined either on the basis of the image or on the basis of the microphone or on both. In a preferred embodiment, the at least one user feature is determined on both or at least comprising at least one user feature based on the image. In a preferred embodiment, the user feature is the combination of the pupil dilation extracted from the images of the second camera 4 taken while the user solves the task and the speech regularity extracted from the audio signal recorded by microphone 5 while the user solves the task. This combined user feature can be for example a two or more dimensional vector comprising one or more dimensions related to the pupil dilation and one or more dimensions related to the speech regularity.

In one embodiment, the user feature means 8 is configured to determine the at least one user feature further based on the positional path of the mobile device 2 while the user solves the task. Preferably, the at least one user feature comprises then at least one first user feature and at least one second user feature. The at least one first user feature is based on the images recorded by the further camera 4 while the user solves the task and/or in the audio signal recorded by the microphone 13 while the user solves the task. The at least one second user feature is based on the positional path of the mobile device 2 while the user solves the task and/or in the audio signal recorded by the microphone 13 while the user solves the task. However, it is also possible to determine a combined user feature which depends on the positional path and of at least one of the images and the audio signal. The at least one (second) user feature based on the positional path of the mobile device 2 could be the momentary variability of the position of the mobile device. This could be for example a frequency or any other measure for this variability.

Preferably, the user feature means 8 is configured to determine a sequence of user feature points during a user feature sampling window. The sequence of user feature points depends on the image points of the image sampling window and/or on the audio points of the audio sampling window. The sequence of user feature points can further depend on the position points of the position sampling window. The user feature sampling window is a time window during which the user solves (at least partly) the task described above. Preferably, the user feature sampling window corresponds to a time window during which the position sampling window and one or both of the image sampling window and the audio sampling window overlaps. The time points to which the user feature points are associated, are called user feature sampling points.

The user feature means 8 is preferably arranged in the mobile device 2. However, it would also be possible to arrange the user feature means 8' completely or partly outside of the mobile device 2, e.g. in the further device 11.

It was found out that not only the movement of the mobile device 2, but also other features of the body of the user during solving the task help significantly to detect the cognitive state of a user.

The cognitive state means 9 is configured to determine a cognitive state of the user based on the positional path of the mobile device 2 determined while the user solves the task and based on the at least one user feature determined while the user solves the task. The cognitive state means 9 is preferably configured to determine a cognitive state of the user based on the positional path of the mobile device 2 (or sequence of position points) in the position sampling window and based on the sequence of the user feature points of the user feature sampling window. The cognitive state means 9 is preferably configured to determine the cognitive state of the user based on measurement points during a measurement sampling window. The measurement points of the measurement sampling window depend on the positional path during the measurement/position sampling window and the images and/or audio signal recorded during the measurement/audio/image sampling window. The measurement points of the measurement sampling window depend on the positional path during the measurement/position sampling window and the user features determined during the measurement/user feature sampling window. The measurement sampling window is a time window during which the user solves (at least partly) the task described above. The time points to which the measurement points are associated, are called measurement sampling points. The measurement sampling points have a measurement sampling frequency and/or measurement sampling steps between two subsequent measurement sampling points. If the user feature points of the user feature window depend already on the position points of the position sampling window, it is also possible that the measurement points of the measurement sampling window and/or the cognitive state are based only the user feature points of the user feature sampling window. The cognitive state means 9 thus receives an input from which it determines the cognitive state of the user. The input of the cognitive state means 9 is:
- the positional path of the mobile device 2 determined while the user solves the task and the at least one user feature determined while the user solves the task,
- the positional path of the mobile device 2 (or sequence of position points) in the position sampling window and the sequence of the user feature points of the user feature sampling window, and/or
- the measurement points during a measurement sampling window. The cognitive state means 9 could extract one or more features from the input data. For example the gait velocity, in particular the stride time variability can be extracted from the positional path of the mobile device 2 for detecting the cognitive state in combination with the user feature. The gait velocity or also called walking frequency can be extracted from the low frequency vertical movements of the mobile device 2. These gait velocity and/or other features extracted from the positional path and/or also the positional path data directly could be used to detect tve state. However, the gait velocity is particularly advantageous as well known in the state of the art. Obviously, it is also possible to detect directly the gait velocity in the path means 6 as the positional path data such that the cognitive state means 9 detects the cognitive state directly on the gait velocity. It is further possible to detect the gait velocity in the user feature means 8.

The determined cognitive state results is preferably one of a number of discrete cognitive states. Preferably, the discrete cognitive states refer to one cognitive feature and the discrete cognitive states correspond to a value or strength of the cognitive feature. Preferably, the cognitive feature or the cognitive state corresponds to a health state of the brain of the user. The health state of the brain of the user refers preferably to one brain disease or one brain damage. Examples for such brain diseases or brain damages are: Alzheimer's disease and/or preclinical Alzheimer's pathological change, frontal temporal dementia, traumatic brain injury, e.g. concussion, Parkinson's, PostOperative Cognitive Disorder, Posterior Cortical Atrophy, Movement Disorders, Neurodegeneration disorders, Cognitive Impairment, Depression. The cognitive state means 9 is then configured to give out one of at least two discrete cognitive states. The at least two discrete cognitive states comprise a first discrete cognitive state stating that the cognitive feature is fulfilled and a second discrete cognitive state stating that the cognitive feature is not fulfilled. Where the cognitive state is a health state, the first discrete cognitive state (first discrete health state) states that the user has the brain disease or the brain damage, and the second discrete cognitive state (second discrete health state) states that the user is healthy with respect to the brain disease or the brain damage. There might be at least one third discrete cognitive/health state stating that the user has a certain risk of fulfilling the cognitive state and/or of having the brain disease or the brain damage.

Preferably, the cognitive state is determined further based on machine learning data. The machine learning data comprises a first condition set for a corresponding first discrete cognitive state. The cognitive state means 9 is preferably configured to check, if the input of the cognitive state means 9 fulfils the first condition set. **If** this is the case, the cognitive state means 9 concludes that the user has a cognitive feature in the first discrete cognitive state. **If** there are more than two discrete cognitive states, other condition sets could be checked for determining the presence of a third or other discrete cognitive state. Otherwise, the user has the second cognitive state. The machine learning data are obtained with machine learning based on user data sets of multiple users. The user data set of each user corresponds to the known cognitive state or health state of the user and the input prepared for the cognitive state means 9 while the task is solved. Preferably, the machine learning data comprise a decision tree. The cognitive state means 9 is configured to go through the decision tree and decide in each node based on the data of the user input to which next node of the decision tree to go. The data of the user comprises at least the positional path of the mobile device 2 and the user features. The data of the user can comprise further user data like age, gender, etc.. The conditions for the decision tree, i.e. the condition sets, are trained in the machine learning phase based on the training data. In one embodiment, at least one node checks at least one feature based on the positional path. One node could be for example check, if the gait velocity (during one or more sub-periods) varies more than a threshold from a reference gait velocity (during a reference sub-period). Different nodes could check the same feature for different sub-periods. The sub-periods refer preferably to sub-periods of time, when the user solves the task, in particular to sub periods where the attention of the user demanded more by the task. These sub-periods are preferably, when the user approaches to the objects he has to search. The reference period is for example a sub-period of the time, during which the user solves the task, in particular a time period which is less attention demanding by the task than the previously mentioned sub periods. Different nodes could check different features of the positional path or of the gait velocity, like its mean, its variance, etc.. In one embodiment, at least one node checks at least one feature based on the user feature, preferably based on the pupil dilation and/or the speech regularity. One node could be for example check, if the user feature (during one or more sub-periods) varies more than a threshold from a reference user feature (during the reference sub-period). Different nodes could check the same feature for different sub-periods. Different nodes could check different features of the user feature, like its mean, its variance, etc.

Preferably, the cognitive state of the user is given out from the apparatus 1. Preferably, the cognitive state of the user is given out via the output means 5 of the mobile device 2.

The cognitive state means 9 is preferably arranged in the further device 11 which communicates with the mobile device 2 to receive the input of the cognitive state means 9. Eventually, the cognitive state means 9 communicates to the mobile device 2 the cognitive state of the user determined such that the mobile device 2 give out the determined cognitive state. However, it would also be possible to arrange the cognitive state means 9' completely or partly in the mobile device 2.

The task provided by the task means 7 asks the attention of the user. The movements of the user, in particular the walking recorded with the path means 6 and the user features recorded with the further camera 4, the microphone 5 and/or with the user feature means 8 during the time when the user attention is taken, i.e. during solving the task, was found to be a reliable and simple indicator for many different cognitive states of the user. This was already known for the movement of the user recorded by the path means 6. However, the results were significantly improved by considering also the user features determined with the user feature means 8.

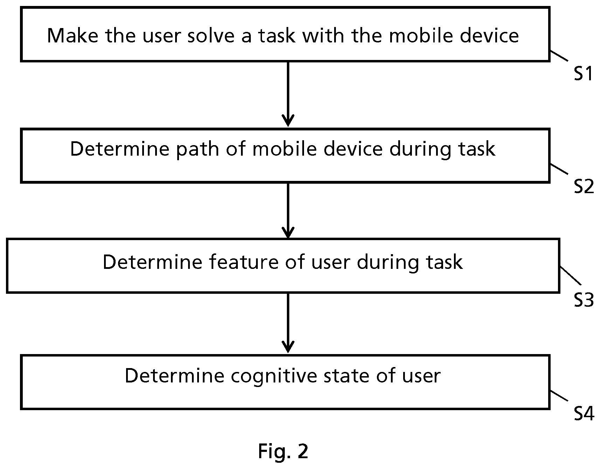

Fig. 2 shows an embodiment for determining a cognitive state of a user.

In step S1, the mobile device 2 invites the user to solve a task. This invitation might be initiated by the mobile device 2. This can be for example be realized by a message displayed on the optical output device 5.1 of the mobile device to solve a certain task. Alternatively, the invitation can be initiated by the user, e.g. by opening an application of the mobile device or by selecting to solve the task. In the preferred task described above, the at least one (preferably at least two) defined target positions are defined by the user in the AR environment of the mobile device 2 and/or are shown to the user in the AR environment of the mobile device 2 (via at least one associated visualization object). Then, the user is invited to search/find the at least one defined target position (e.g. by showing the visualization object associated with the defined target position to be searched).

In step S2, the path of the mobile device 2 is recorded, while (at least a part of) the task is solved.

In step S3, the images of the second camera 4 and/or the audio signal of the microphone 4 is recorded, while (at least a part of) the task is solved. At least one user feature of the user is determined based on the images and/or the audio signal recorded, while (at least a part of) the task is solved.

The steps S1 and the recording of the positional path of S2, the images and/or the audio signal of step S3 are performed preferably contemporaneously (i.e. at least the measurements of the steps S2 and S3 are timewise at least partially overlapping with step S1).

In step S4, the cognitive state of the user is determined based on an input which is based on the positional path of the mobile device 2 determined in step S2 and on the at least one feature of the user determined in step S3.

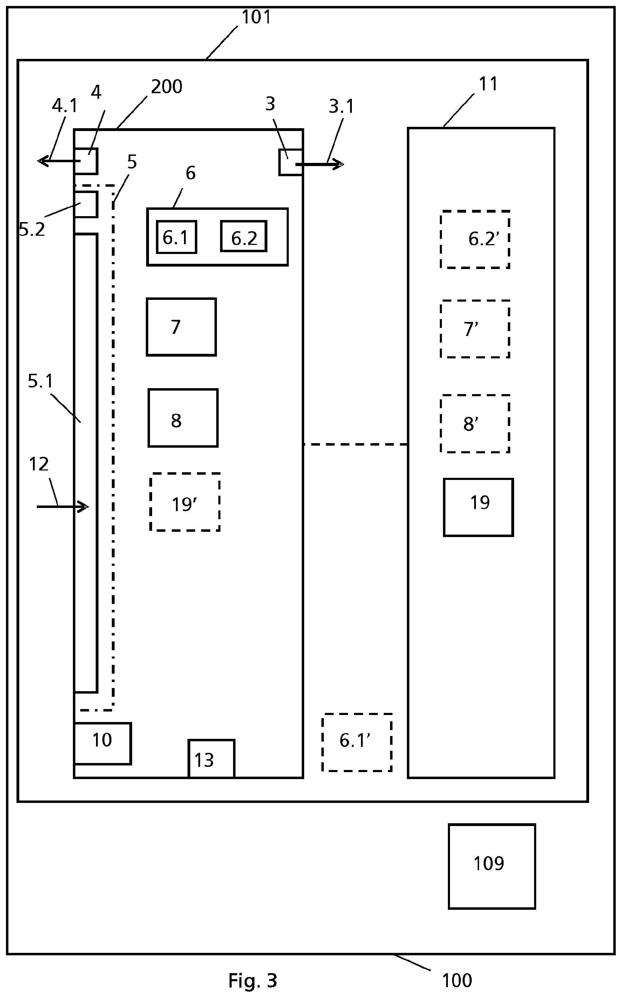

Fig. 3 shows an apparatus 100 for determining machine learning data for determining a cognitive state of a user. The apparatus 100 comprises at least one apparatus 101 for determining the input defined above for the cognitive state means 9 of the apparatus 1. Therefore, the apparatus 101 comprises a mobile device 200, a first camera in the mobile device 200 working as the first camera 3 in the mobile device 2, a second camera and/or a microphone 13 in the mobile device 200 working as the second camera 4 and/or the microphone 13 in the mobile device 2, an output means 5 in the mobile device 200 working as the output means 5 in the mobile device 2, a path means 6 working as the path means 6, a task means 7 working as the task means 7 in Fig. 1, a user feature means 8 working as the user feature means 8 in Fig. 1, preferably a user input means 10 working as the user input means 10 in Fig. 1 and an input preparation means 19 or 19' instead of the cognitive state means 9. The input preparation means is configured to prepare the input which is defined above and/or which is input to the cognitive state means 9 of the apparatus 1. The input is based on the positional path of the mobile device determined while the user solves the task and the at least one user feature determined while the user solves the task.

The apparatus 101 corresponds preferably to the apparatus 1 of Fig. 1 with two configurations. A first configuration for determining a cognitive state of a user as described in Fig. 1. A second configuration for determining the input for a user as described in the previous paragraph.

The apparatus 100 comprises further the machine learning data means 109 which is configured to receive for each of multiple (preferably different) users a user data set. The user data set of a user comprises the cognitive state of the user and the input created in one of the at least one apparatus 101, while the user solved the task. The machine learning data means 109 is further configured to determine the machine learning data for determining a cognitive state on the input for a user based on the user data set for the multiple users. In one embodiment, the machine learning data means 109 receives for all users the user data sets and determines the machine learning data (only once). In one embodiment, the machine learning data means 109 is configured to update the machine learning data each time it receives user data set(s) of one or another number defined number of user(s). In this embodiment, the machine learning data are determined recursively. Preferably, the machine learning data means 109 is configured to determine the decision parameters of a decision tree based on the user data set(s). Preferably, the machine learning data means 109 is configured to determine the machine learning data based on boosting.

The machine learning data means 109 is preferably arranged in the further device 11. However, the machine learning data means 109 can be (partly or completely) in the mobile device 200 or in another device.

## Claims

1. Apparatus for determining a cognitive state of a user of a mobile device (2) wherein the cognitive state corresponds to a health state of the brain of the user, the apparatus (1) comprising:
the mobile device (2) comprising:
a first camera (3) arranged to record images in a first view direction (3.1) of an environment of the mobile device (2);
a second camera (4) configured for recording images in a second view direction (4.1) and configured to record images of at least one feature of the user, and/or a microphone (13) configured for recording an audio signal; and
optical output means (5.1) configured for giving out optical information to the user;
the apparatus further comprising:
a path means (6) configured to determine a positional path of the mobile device (2) in the environment;
a task means (7, 7') configured for interacting via the output means (5) with the user to make the user solve a task using an augmented reality environment shown via the optical output means (5.1) and based on the environment of the mobile device (2) captured by the first camera (3), wherein the task means is further configured to interact via the mobile phone with the user to make the user solve a distracting sub-task while the user is solving the task;
a cognitive state means (9, 9') for determining the cognitive state of the user based on the positional path of the mobile device determined while the user solves the task;
a user feature means (8, 8') for detecting and/or tracking at least one feature of the user in the images recorded by the second camera (4) while the user solves the task and/or in the audio signal recorded by the microphone (13) while the user solves the task;
wherein the cognitive state means (9, 9') is configured to determine the cognitive state of user further based on the at least one feature of the user.

2. Apparatus according to claim 1, wherein the second camera (4) is configured to take a series of images while the user solves the task, wherein the user feature means (8, 8') is configured to detect the at least one feature of the user in the series of images and to determine a time series of the at least one feature detected in the series of images, wherein the cognitive state means (9, 9') is configured to determine the cognitive state of user further based on the time series of the at least one feature of the user.

3. Apparatus according to claim 1 or 2, wherein the at least one feature of the user detected and/or tracked in the images recorded by the second camera (4) while the user solves the task comprises a pupil dilation of an eye of the user.

4. Apparatus according to any preceding claim, wherein a sampling window comprises a plurality of sampling steps over time, wherein the path means (6) is configured record in each sampling step at least one position of the mobile device (2), wherein the second camera (4) is configured to record at least one image in each sampling step, wherein the user feature means (8, 8') is configured to detect for each sampling step the at least one feature of the user in the at least one image of recorded in the sampling step by the second camera (4), wherein the cognitive state means (9, 9') is configured to determine the cognitive state of user based on a time series in the sampling window comprising for each sampling step a sampling point based on the at least one feature of the user detected in this sampling step and based on the at least one position of the mobile device (2) determined in this sampling step.

5. Apparatus according to any preceding claim, wherein the optical output means (5.1) comprises a display arranged on a first side of the mobile device (2), wherein the second camera (4) is arranged on the first side of the mobile device (2) and/or is arranged such that the second view direction shows in the same direction as the normal vector of surface of the display, wherein the camera (3) is arranged on a second side of the mobile device (2) opposed to the first side and/or is arranged such that the first view direction shows in the opposite direction as the normal vector of surface of the display.

6. Apparatus according to any preceding claim,
wherein the task means (7) is configured to interact via the output means (5) with the user to make the user walk with the mobile device (2) in the environment.

7. Apparatus according to any preceding claim,
wherein the task means (7) is configured to define at least one target position in the augmented reality environment and to perform the following steps for each of the at least one target position :
invite the user to find the respective target position in the augmented reality environment;
consider the respective target position as found in the augmented reality environment, when the user has moved the mobile device (2) in a position in the environment, in which the at least one target position is captured by the camera (3) and is shown, maybe under certain conditions, on the optical output means (5.1).

8. Apparatus according to claim 7, wherein the task means (7, 7') is configured to define the at least one target position in the augmented reality environment based on a user input of the mobile device (2).

9. Apparatus according to claim 7 or 8, wherein the at least one target position comprises at least two target positions, wherein the task means (7, 7') is configured
to associate each of the at least two target positions with a different feature which is output to the user by the output means (5), when the respective target position is defined and/or shown in the augmented reality environment, wherein the user is invited to find one of the at least two target positions in the augmented reality environment by outputting the feature associated with the one target position by the output means (5), and /or
to subsequently invite the user to find the at least two target positions such that after one of the at least two target positions is considered to be found, the user is invited to find a subsequent one of the at least two target positions.

10. Apparatus according to any preceding claim, wherein the task means (7, 7') is configured to give out via the output means (5) a plurality of stimuli for distracting the user while solving the task and/or while finding each of the at least one target positions.

11. Apparatus according claim 10, wherein the task means (7, 7') is configured to require the user after each stimulus to solve the sub-task before continuing with solving the task and/or with finding the respective target position invited to find.

12. Apparatus according to claim 11, comprising a user input means (10), wherein the task means (7, 7') is configured to consider the sub-task solved, when receiving a certain user input over the user input means (10).

13. Apparatus according to any preceding claim, wherein the cognitive state is a health state with respect to at least one brain disease or brain damage.

14. A computer program configured to cause an apparatus according to any preceding claim to determine a cognitive state of a user of a mobile device (2) wherein the cognitive state corresponds to a health state of the brain of the user, the computer program being configured:
to cause the first camera (3) to record images in the first view direction (3.1) of an environment of the mobile device (2) ;
to cause the second camera (4) to record images in the second view direction (4.1) and to record images of at least one feature of the user, and/or to cause a microphone (13) to record an audio signal;
to cause the optical output means (5.1) to give out optical information to the user;
wherein the computer program further causes:
the path means (6) of the apparatus to determine a positional path of the mobile device (2) in the environment;
the task means (7, 7') of the apparatus to interact via the output means (5) with the user to make the user solve a task using an augmented reality environment shown via the optical output means (5.1) and based on the environment of the mobile device (2) captured by the first camera (3),
the task means further to interact via the mobile phone with the user to make the user solve a distracting sub-task while the user is solving the task;
the cognitive state means (9, 9') of the apparatus to determine the cognitive state of the user based on the positional path of the mobile device determined while the user solves the task;
the user feature means (8, 8') of the apparatus to detect and/or track at least one feature of the user in the images recorded by the second camera (4) while the user solves the task and/or in the audio signal recorded by the microphone (13) while the user solves the task; and
the cognitive state means (9, 9') to determine the cognitive state of user further based on the at least one feature of the user.

## Patentansprüche

1. Vorrichtung zur Bestimmung eines kognitiven Zustands eines Benutzers eines mobilen Geräts (2), wobei der kognitive Zustand einem Gesundheitszustand des Gehirns des Benutzers entspricht, wobei die Vorrichtung (1) umfasst:
das mobile Gerät (2) umfasst:
eine erste Kamera (3), die so angeordnet ist, dass sie Bilder in einer ersten Blickrichtung (3.1) einer Umgebung des mobilen Geräts (2) aufzeichnet;
eine zweite Kamera (4), die zum Aufzeichnen von Bildern in einer zweiten Blickrichtung (4.1) konfiguriert ist und zum Aufzeichnen von Bildern von mindestens einem Merkmal des Benutzers konfiguriert ist, und/oder ein Mikrofon (13), das zum Aufzeichnen eines Audiosignals konfiguriert ist; und
optische Ausgabemittel (5.1), die zum Ausgeben optischer Informationen an den Benutzer konfiguriert sind;
die Vorrichtung 1 umfasst weiter:
eine Wegeeinrichtung (6), das so konfiguriert ist, dass es einen Positionsweg des mobilen Geräts (2) in der Umgebung bestimmt;
einen Aufgabenmittel (7, 7'), die so konfiguriert ist, dass sie über die Ausgabemittel (5) mit dem Benutzer interagiert, um den Benutzer dazu zu bringen, eine Aufgabe unter Verwendung einer Augmented-Reality-Umgebung, die über die optische Ausgabeeinrichtung (5.1) angezeigt wird und auf der Umgebung des mobilen Geräts (2) basiert, die von der ersten Kamera (3) erfasst wurde, wobei das Aufgabenmittel ferner so konfiguriert ist, dass es über das Mobiltelefon mit dem Benutzer interagiert, um den Benutzer dazu zu bringen, eine ablenkende Teilaufgabe zu lösen, während der Benutzer die Aufgabe löst;
ein kognitives Zustandsmittel (9, 9') zum Bestimmen des kognitiven Zustands des Benutzers auf der Grundlage des Positionspfads des mobilen Geräts, der bestimmt wird, während der Benutzer die Aufgabe löst;
ein Benutzermerkmalmittel (8, 8') zum Erfassen und/oder Verfolgen mindestens eines Merkmals des Benutzers in den von der zweiten Kamera (4) aufgezeichneten Bildern, während der Benutzer die Aufgabe löst, und/oder in dem vom Mikrofon (13) aufgezeichneten Audiosignal, während der Benutzer die Aufgabe löst;
wobei das kognitive Zustandsmittel (9, 9') so konfiguriert sind, dass es den kognitiven Zustand des Benutzers weiter auf der Grundlage des mindestens einen Merkmals des Benutzers bestimmt.

2. Vorrichtung nach Anspruch 1, wobei die zweite Kamera (4) so konfiguriert ist, dass sie eine Reihe von Bildern aufnimmt, während der Benutzer die Aufgabe löst, wobei das Benutzermerkmalsmittel (8, 8') so konfiguriert ist, dass es das mindestens ein Merkmal des Benutzers in der Reihe von Bildern erkennt und eine Zeitreihe des mindestens einen in der Reihe von Bildern erkannten Merkmals bestimmt, wobei das kognitive Zustandsmittel (9, 9') so konfiguriert ist, dass es den kognitiven Zustand des Benutzers auf der Grundlage der Zeitreihe des mindestens einen Merkmals des Benutzers weiter bestimmt.

3. Vorrichtung nach Anspruch 1 oder 2, wobei das mindestens eine Merkmal des Benutzers, das in den von der zweiten Kamera (4) aufgezeichneten Bildern erkannt und/oder verfolgt wird, während der Benutzer die Aufgabe löst, eine Pupillenerweiterung eines Auges des Benutzers umfasst.

4. Vorrichtung nach einem der vorstehenden Ansprüche, wobei ein Abtastfenster eine Vielzahl von Abtastschritten über die Zeit umfasst, wobei die Wegeeinrichtung (6) so konfiguriert ist, dass sie in jedem Abtastschritt mindestens eine Position des mobilen Geräts (2) aufzeichnet, wobei die zweite Kamera (4) so konfiguriert ist, dass sie in jedem Abtastschritt mindestens ein Bild aufzeichnet, wobei das Benutzermerkmalmittel (8, 8') so konfiguriert ist, dass es für jeden Abtastschritt das mindestens eine Merkmal des Benutzers in dem mindestens einen Bild erfasst, das in dem Abtastschritt von der zweiten Kamera (4) aufgezeichnet wurde, wobei das kognitive Zustandsmittel (9, 9') so konfiguriert ist, dass es den kognitiven Zustand des Benutzers auf der Grundlage einer Zeitreihe im Abtastfenster bestimmt, die für jeden Abtastschritt einen Abtastpunkt auf der Grundlage des mindestens einen in diesem Abtastschritt erkannten Merkmals des Benutzers und auf der Grundlage der mindestens einen in diesem Abtastschritt bestimmten Position des mobilen Geräts (2) umfasst.

5. Vorrichtung nach einem der vorstehenden Ansprüche, wobei das optische Ausgabemittel (5.1) eine auf einer ersten Seite des mobilen Geräts (2) angeordnete Anzeige umfasst, wobei die zweite Kamera (4) auf der ersten Seite des mobilen Geräts (2) angeordnet ist und/oder so angeordnet ist, dass die zweite Blickrichtung zeigt in die gleiche Richtung wie der Normalenvektor der Oberfläche der Anzeige zeigt, wobei die Kamera (3) auf einer der ersten Seite gegenüberliegenden zweiten Seite des mobilen Geräts (2) angeordnet ist und/oder so angeordnet ist, dass die erste Blickrichtung in die entgegengesetzte Richtung wie der Normalenvektor der Oberfläche der Anzeige zeigt.

6. Vorrichtung nach einem der vorstehenden Ansprüche,
wobei das Aufgabenmittel (7) so konfiguriert ist, über die Ausgabemittel (5) mit dem Benutzer zu interagieren, um den Benutzer dazu zu bringen, mit dem mobilen Gerät (2) in der Umgebung zu gehen.

7. Vorrichtung nach einem der vorstehenden Ansprüche,
wobei das Aufgabenmittel (7) so konfiguriert ist, dass es mindestens eine Zielposition in der Augmented-Reality-Umgebung definieren und für jede der mindestens einen Zielposition die folgenden Schritte ausführen
den Benutzer aufzufordern, die jeweilige Zielposition in der Augmented-Reality-Umgebung zu finden;
die jeweilige Zielposition, wie sie in der Augmented-Reality-Umgebung gefunden wird, zu berücksichtigen, wenn der Benutzer das mobile Gerät (2) in eine Position in der Umgebung bewegt hat, in der die mindestens eine Zielposition von der Kamera (3) erfasst und, möglicherweise unter bestimmten Bedingungen, auf dem optischen Ausgabemittel (5.1) angezeigt wird.

8. Vorrichtung nach Anspruch 7, wobei das Aufgabenmittel (7, 7') so konfiguriert ist, dass sie die mindestens eine Zielposition in der Augmented-Reality-Umgebung auf der Grundlage einer Benutzereingabe des mobilen Geräts (2) definiert.

9. Vorrichtung nach Anspruch 7 oder 8, wobei die mindestens eine Zielposition mindestens zwei Zielpositionen umfasst, wobei das Aufgabenmittel (7, 7') so konfiguriert ist, jede der mindestens zwei Zielpositionen mit einem unterschiedlichen Merkmal zu verknüpfen, das dem Benutzer durch das Ausgabemittel (5) ausgegeben wird, wenn die jeweilige Zielposition in der Augmented-Reality-Umgebung definiert und/oder angezeigt wird, wobei der Benutzer aufgefordert wird, eine der mindestens zwei Zielpositionen in der Augmented-Reality-Umgebung zu finden, indem das mit der einen Zielposition assoziierte Merkmal durch das Ausgabemittel (5) ausgegeben wird, und/oder
um anschließend den Benutzer aufzufordern, die mindestens zwei Zielpositionen zu finden,
sodass der Benutzer, nachdem eine der mindestens zwei Zielpositionen als gefunden gilt, aufgefordert wird, eine weitere der mindestens zwei Zielpositionen zu finden.

10. Vorrichtung nach einem der vorstehenden Ansprüche, wobei das Aufgabenmittel (7, 7') so konfiguriert ist, dass es über das Ausgabemittel (5) eine Vielzahl von Reizen ausgibt, um den Benutzer während der Lösung der Aufgabe und/oder während des Findens jeder der mindestens einen Zielposition abzulenken.

11. Vorrichtung nach Anspruch 10, wobei das Aufgabenmittel (7, 7') so konfiguriert ist, dass es vom Benutzer nach jedem Reiz verlangt, die Teilaufgabe zu lösen, bevor er mit der Lösung der Aufgabe und/oder mit dem Auffinden der jeweiligen Zielposition, die er finden soll, fortfahren kann.

12. Vorrichtung nach Anspruch 11, mit einer Benutzereingabeeinrichtung (10), wobei das Aufgabenmittel (7, 7') so konfiguriert ist, dass es die Teilaufgabe als gelöst betrachtet, wenn es eine bestimmte Benutzereingabe über die Benutzereingabevorrichtung (10) empfängt.

13. Vorrichtung nach einem der vorstehenden Ansprüche, wobei der kognitive Zustand ein Gesundheitszustand in Bezug auf mindestens eine Hirnerkrankung oder Hirnschädigung ist.

14. Ein Computerprogramm, das so konfiguriert ist, dass es eine Vorrichtung gemäß einem der vorstehenden Ansprüche veranlasst, einen kognitiven Zustand eines Benutzers eines mobilen Geräts (2) zu bestimmen, wobei der kognitive Zustand einem Gesundheitszustand des Gehirns des Benutzers entspricht, wobei das Computerprogramm so konfiguriert ist, dass es
die erste Kamera (3) dazu veranlasst, Bilder in der ersten Blickrichtung (3.1) einer Umgebung des mobilen Geräts (2) aufzunehmen;
die zweite Kamera (4) dazu veranlasst, Bilder in der zweiten Blickrichtung (4.1) aufzunehmen und Bilder von mindestens einem Merkmal des Benutzers aufzunehmen, und/oder ein Mikrofon (13) dazu veranlassen, ein Audiosignal aufzunehmen;
das optische Ausgabemittel (5.1) dazu veranlasst, optische Informationen an den Benutzer auszugeben;
wobei das Computerprogramm weiter veranlasst, dass
die Wegeeinrichtung (6) der Vorrichtung einen Positionsweg des mobilen Geräts (2) in der Umgebung bestimmt;
das Aufgabenmittel (7, 7') der Vorrichtung über die Ausgabemittel (5) mit dem Benutzer interagiert, um den Benutzer dazu zu bringen, eine Aufgabe unter Verwendung einer über das optischen Ausgabemittel (5.1) angezeigten Augmented-Reality-Umgebung und auf der Grundlage der von der ersten Kamera (3) erfassten Umgebung des mobilen Geräts (2) zu lösen
das Aufgabenmittel ferner über das Mobiltelefon mit dem Benutzer zu interagieren, um den Benutzer dazu zu bringen, eine ablenkende Teilaufgabe zu lösen, während der Benutzer die Aufgabe löst;
das kognitive Zustandsmittel (9, 9') der Vorrichtung, um den kognitiven Zustand des Benutzers auf der Grundlage des Positionspfads des mobilen Geräts zu bestimmen, der ermittelt wird, während der Benutzer die Aufgabe löst;
die Benutzermerkmalmittel (8, 8') der Vorrichtung zum Erfassen und/oder Verfolgen mindestens eines Merkmals des Benutzers in den von der zweiten Kamera (4) aufgezeichneten Bildern, während der Benutzer die Aufgabe löst, und/oder in dem vom Mikrofon (13) aufgezeichneten Audiosignal, während der Benutzer die Aufgabe löst; und
das kognitive Zustandsmittel (9, 9'), um den kognitiven Zustand des Benutzers weiter auf der Grundlage des mindestens einen Merkmals des Benutzers zu bestimmen.

## Revendications

1. - Appareil permettant de déterminer un état cognitif d'un utilisateur d'un dispositif mobile (2), dans lequel l'état cognitif correspond à un état de santé du cerveau de l'utilisateur, l'appareil (1) comprenant :
le dispositif mobile (2) comprenant :
une première caméra (3) agencée pour enregistrer des images dans une première direction de visualisation (3.1) d'un environnement du dispositif mobile (2) ;
une seconde caméra (4) configurée pour enregistrer des images dans une seconde direction de visualisation (4.1) et configurée pour enregistrer des images d'au moins une caractéristique de l'utilisateur, et/ou un microphone (13) configuré pour enregistrer un signal audio ; et
un moyen de sortie optique (5.1) configuré pour transmettre des informations optiques à l'utilisateur ;
l'appareil comprenant en outre :
un moyen de chemin (6) configuré pour déterminer un chemin de position du dispositif mobile (2) dans l'environnement ;
un moyen de tâche (7, 7') configuré pour interagir par l'intermédiaire du moyen de sortie (5) avec l'utilisateur afin d'amener l'utilisateur à résoudre une tâche à l'aide d'un environnement de réalité augmentée représenté par l'intermédiaire du moyen de sortie optique (5.1) et sur la base de l'environnement du dispositif mobile (2) capturé par la première caméra (3), le moyen de tâche étant en outre configuré pour interagir par l'intermédiaire du téléphone mobile avec l'utilisateur afin d'amener l'utilisateur à résoudre une sous-tâche de distraction pendant que l'utilisateur résout la tâche ;
un moyen d'état cognitif (9, 9') pour déterminer l'état cognitif de l'utilisateur sur la base du chemin de position du dispositif mobile déterminé pendant que l'utilisateur résout la tâche ;
un moyen de caractéristique d'utilisateur (8, 8') pour détecter et/ou suivre au moins une caractéristique de l'utilisateur dans les images enregistrées par la seconde caméra (4) pendant que l'utilisateur résout la tâche et/ou dans le signal audio enregistré par le microphone (13) pendant que l'utilisateur résout la tâche ;
dans lequel le moyen d'état cognitif (9, 9') est configuré pour déterminer l'état cognitif de l'utilisateur en outre sur la base de l'au moins une caractéristique de l'utilisateur.

2. - Appareil selon la revendication 1, dans lequel la seconde caméra (4) est configurée pour prendre une série d'images pendant que l'utilisateur résout la tâche, dans lequel le moyen de caractéristique d'utilisateur (8, 8') est configuré pour détecter l'au moins une caractéristique de l'utilisateur dans la série d'images et pour déterminer une série temporelle de l'au moins une caractéristique détectée dans la série d'images, dans lequel le moyen d'état cognitif (9, 9') est configuré pour déterminer l'état cognitif de l'utilisateur en outre sur la base de la série temporelle de l'au moins une caractéristique de l'utilisateur.

3. - Appareil selon la revendication 1 ou 2, dans lequel l'au moins une caractéristique de l'utilisateur détectée et/ou suivie dans les images enregistrées par la seconde caméra (4) pendant que l'utilisateur résout la tâche comprend une dilatation de la pupille d'un œil de l'utilisateur.

4. - Appareil selon l'une quelconque des revendications précédentes, dans lequel une fenêtre d'échantillonnage comprend une pluralité d'étapes d'échantillonnage au cours du temps, dans lequel le moyen de chemin (6) est configuré pour enregistrer dans chaque étape d'échantillonnage au moins une position du dispositif mobile (2), dans lequel la seconde caméra (4) est configurée pour enregistrer au moins une image dans chaque étape d'échantillonnage, dans lequel le moyen de caractéristique d'utilisateur (8, 8') est configuré pour détecter pour chaque étape d'échantillonnage l'au moins une caractéristique de l'utilisateur dans l'au moins une image enregistrée dans l'étape d'échantillonnage par la seconde caméra (4), dans lequel le moyen d'état cognitif (9, 9') est configuré pour déterminer l'état cognitif de l'utilisateur sur la base d'une série temporelle dans la fenêtre d'échantillonnage comprenant pour chaque étape d'échantillonnage un point d'échantillonnage sur la base de l'au moins une caractéristique de l'utilisateur détectée dans cette étape d'échantillonnage et sur la base de l'au moins une position du dispositif mobile (2) déterminée dans cette étape d'échantillonnage.

5. - Appareil selon l'une quelconque des revendications précédentes, dans lequel le moyen de sortie optique (5.1) comprend un dispositif d'affichage disposé sur un premier côté du dispositif mobile (2), dans lequel la seconde caméra (4) est disposée sur le premier côté du dispositif mobile (2) et/ou est disposée de telle sorte que la seconde direction de visualisation indique la même direction que le vecteur normal de surface du dispositif d'affichage, dans lequel la caméra (3) est disposée sur un second côté du dispositif mobile (2) opposé au premier côté et/ou est disposée de telle sorte que la première direction de visualisation indique la direction opposée au vecteur normal de surface du dispositif d'affichage.

6. - Appareil selon l'une quelconque des revendications précédentes, dans lequel le moyen de tâche (7) est configuré pour interagir par l'intermédiaire du moyen de sortie (5) avec l'utilisateur pour amener l'utilisateur à marcher avec le dispositif mobile (2) dans l'environnement.

7. - Appareil selon l'une quelconque des revendications précédentes, dans lequel le moyen de tâche (7) est configuré pour définir au moins une position cible dans l'environnement de réalité augmentée et pour effectuer les étapes suivantes pour chacune de l'au moins une position cible :
inviter l'utilisateur à trouver la position cible respective dans l'environnement de réalité augmentée ;
considérer la position cible respective comme trouvée dans l'environnement de réalité augmentée, lorsque l'utilisateur a déplacé le dispositif mobile (2) dans une position dans l'environnement, dans laquelle l'au moins une position cible est capturée par la caméra (3) et est montrée, le cas échéant sous certaines conditions, sur le moyen de sortie optique (5.1).

8. - Appareil selon la revendication 7, dans lequel le moyen de tâche (7, 7') est configuré pour définir l'au moins une position cible dans l'environnement de réalité augmentée sur la base d'une entrée d'utilisateur du dispositif mobile (2).

9. - Appareil selon la revendication 7 ou 8, dans lequel l'au moins une position cible comprend au moins deux positions cibles, dans lequel le moyen de tâche (7, 7') est configuré
pour associer chacune des au moins deux positions cibles à une caractéristique différente qui est transmise à l'utilisateur par le moyen de sortie (5), lorsque la position cible respective est définie et/ou montrée dans l'environnement de réalité augmentée, l'utilisateur étant invité à trouver l'une des au moins deux positions cibles dans l'environnement de réalité augmentée en transmettant la caractéristique associée à ladite position cible par le moyen de sortie (5), et/ou
pour inviter ultérieurement l'utilisateur à trouver les au moins deux positions cibles de telle sorte qu'après que l'une des au moins deux positions cibles est considérée comme étant trouvée, l'utilisateur est invité à trouver une position suivante des au moins deux positions cibles.

10. - Appareil selon l'une quelconque des revendications précédentes, dans lequel le moyen de tâche (7, 7') est configuré pour transmettre par l'intermédiaire du moyen de sortie (5) une pluralité de stimuli pour distraire l'utilisateur pendant qu'il résout la tâche et/ou pendant qu'il trouve chacune de l'au moins une position cible.

11. - Appareil selon la revendication 10, dans lequel le moyen de tâche (7, 7') est configuré pour demander à l'utilisateur, après chaque stimulus, de résoudre la sous-tâche avant de continuer à résoudre la tâche et/ou à trouver la position cible respective qu'il est invité à trouver.

12. - Appareil selon la revendication 11, comprenant un moyen d'entrée d'utilisateur (10), dans lequel le moyen de tâche (7, 7') est configuré pour considérer la sous-tâche résolue, lorsqu'il reçoit une certaine entrée d'utilisateur sur le moyen d'entrée d'utilisateur (10).

13. - Appareil selon l'une quelconque des revendications précédentes, dans lequel l'état cognitif est un état de santé par rapport à au moins une maladie du cerveau ou une lésion cérébrale.

14. - Programme d'ordinateur configuré pour amener un appareil selon l'une quelconque des revendications précédentes à déterminer un état cognitif d'un utilisateur d'un dispositif mobile (2), dans lequel l'état cognitif correspond à un état de santé du cerveau de l'utilisateur, le programme d'ordinateur étant configuré :
pour amener la première caméra (3) à enregistrer des images dans la première direction de visualisation (3.1) d'un environnement du dispositif mobile (2) ;
pour amener la seconde caméra (4) à enregistrer des images dans la seconde direction de visualisation (4.1) et à enregistrer des images d'au moins une caractéristique de l'utilisateur, et/ou à amener un microphone (13) à enregistrer un signal audio ;
pour amener le moyen de sortie optique (5.1) à transmettre des informations optiques à l'utilisateur ;
dans lequel le programme d'ordinateur amène en outre :
le moyen de chemin (6) de l'appareil à déterminer un chemin de position du dispositif mobile (2) dans l'environnement ;
le moyen de tâche (7, 7') de l'appareil à interagir par l'intermédiaire du moyen de sortie (5) avec l'utilisateur pour amener l'utilisateur à résoudre une tâche à l'aide d'un environnement de réalité augmentée représenté par l'intermédiaire du moyen de sortie optique (5.1) et sur la base de l'environnement du dispositif mobile (2) capturé par la première caméra (3) ;
le moyen de tâche en outre à interagir par l'intermédiaire du téléphone mobile avec l'utilisateur pour amener l'utilisateur à résoudre une sous-tâche de distraction pendant que l'utilisateur résout la tâche ;
le moyen d'état cognitif (9, 9') de l'appareil à déterminer l'état cognitif de l'utilisateur sur la base du chemin de position du dispositif mobile déterminé pendant que l'utilisateur résout la tâche ;
le moyen de caractéristique d'utilisateur (8, 8') de l'appareil à détecter et/ou à suivre au moins une caractéristique de l'utilisateur dans les images enregistrées par la seconde caméra (4) pendant que l'utilisateur résout la tâche et/ou dans le signal audio enregistré par le microphone (13) pendant que l'utilisateur résout la tâche ; et
le moyen d'état cognitif (9, 9') à déterminer l'état cognitif de l'utilisateur en outre sur la base de l'au moins une caractéristique de l'utilisateur.
